Europäisches Patentamt

European Patent Office

Office européen des brevets

(19)

(11) Publication number : **0 466 462 A1**

(12) **EUROPEAN PATENT APPLICATION**

(21) Application number : **91306228.7**

(22) Date of filing : **09.07.91**

(51) Int. Cl.⁵ : **A61M 1/06**

(30) Priority : **10.07.90 US 550619**
**01.02.91 US 649108**

(43) Date of publication of application :
**15.01.92 Bulletin 92/03**

(84) Designated Contracting States :
**DE GB**

(71) Applicant : **I S G AG**
**Alpenstrasse 2**
**CH-6300 Zug (CH)**

(72) Inventor : **Larsson, Karl Olof Axel Helmer**
**Lindenweg 2**
**CH-6300 Zug (CH)**

(74) Representative : **Alexander, Thomas Bruce et al**
**Boult, Wade & Tennant 27 Furnival Street**
**London EC4A 1PQ (GB)**

(54) **Breast pump with massage for improved lactation.**

(57)  A breast pump is disclosed having a hood with breast massage. In one embodiment, a frame 18 supports the hood 1, substantially preventing contraction of the hood along its longitudinal axis, while permitting a thin-walled portion 5 to contract along its diameter. Under action of a suction pump, the flexible thin-walled portion gently massages the areola. Another embodiment has a flexible sleeve within which the nipple is received. An exterior frame again provides support for the hood, allowing the sleeve to radially contract under suction to gently massage the nipple. The frame prevents the longitudinal contraction of the sleeve that could pinch the nipple and breast.

FIG. 2

## FIELD OF INVENTION

The present invention generally relates to a breast pump for the expression of mother's milk. More particularly, the invention relates to a new and improved suction hood for use in a breast pump wherein the hood receives a mother's breast and provides a combination of suction and gentle massage of the nipple and/or areola for better and more natural lactation.

## BACKGROUND OF THE INVENTION

Breast pumps for the expression of mothers' milk are well known in the art. These pumps typically have a funnel shaped hood that fits over the breast. The hood is in communication with either a motor driven or manually operated vacuum pump. The vacuum pump generates an intermittent vacuum, or suction, within the hood which pulls the breast into the narrowed neck of the hood, thereby causing the expression of milk. An example of such a pump is seen in applicant's U.S. Patent No. 4,857,051.

It is considered desirable for the hood to create a suckling effect to stimulate lactation, while still providing comfort to the mother. To achieve such an effect, the hood can be provided with a flexible area about the nipple as well as areola which contracts to compress and gently massage the breast. In addition to maintaining flexibility, however, it is important that the hood be supported sufficiently to prevent collapse of the hood around the breast, and particularly the nipple, which could cause discomfort, e.g., pinching, and also inhibit milk flow.

Various hood constructions have been disclosed in an attempt to achieve breast massage during lactation. For instance, breast pumps have been disclosed with flexible reduced-thickness areas in the portion of the hood or breast receptor wall around the nipple area. Stuart, U.S. Patent No. 4,680,028, shows a hood or breast receptor "in which the merging portions of the frusto-conical walls of the skirt (or hood) and the wall of the tubular stem (extending downstream from the skirt) are relatively thin compared with the remaining wall portions of the receptor." In Beer et al., U.S. Patent No. 4,799,922, a flexible insert is disclosed which is adapted to fit within a rigid hood on a breast pump. The insert has a "deformation zone" near the point of contact with the nipple in which the wall thickness is reduced to increase flexibility for a maximum suckling effect.

Varying wall thickness in the wall of the hood at or near the nipple area to achieve flexibility for nipple massage is one attempt to restrain total collapse of the hood, resulting in pinching of the nipple, which would be both uncomfortable to the mother and would inhibit milk flow. These hoods have had to compromise on the length of the stroking zone, however, by maintaining a thickened wall overall in an attempt to prevent axial contraction of the flexible hood.

The Stuart breast receptor maintains a thickened and thus less flexible wall overall (0.077 inches at its thinnest point). Beer et al. also recognizes the need to prevent collapse of the flexible insert, and provides for a wall thickness ratio and a thickened helmet structure on the "thimble" end of the insert such that the suction from a pump "should cause no significant deformation of the thimble in transverse (i.e., radial) direction either."

Both Stuart and Beer et al. also have a contracted or narrowed portion near the hood outlet forming an interior shoulder, which creates an area for milk to collect during operation of the breast pump. A smoothly tapering funnel for the hood extending into a substantially constant diameter flexible sleeve would prevent any milk from pooling in the hood.

It would be desirable to have a breast pump with a flexible areola and/or nipple massaging area which is of increased length for a longer area of stimulation, so as to accommodate breasts and nipples in a wide range of sizes and shapes, yet which will not significantly contract along its longitudinal axis, to thereby avoid pinching. Such a pump would achieve better stimulation of the suckling effect, with increased lactation and comfort for the mother.

## SUMMARY OF THE INVENTION

It is a principal objective of this invention to provide a breast pump that has an improved hood structure which will stimulate a desired part of the breast tip area constituting the nipple and areola by gentle massage encouraging enhanced lactation while maintaining the comfort of the mother. It is a further objective of the invention to provide a flexible breast pump hood structure which has a widened flexible or resilient area that will contract radially along its diameter to provide nipple and/or areola stimulation in the form of a gentle touching of the nipple and/or areola, and that will not collapse along its axis (in a longitudinal direction), which could cause discomfort to the nipple and inhibit milk flow.

To these and other ends, the present invention comprises a generally funnel-shaped hood for receiving a substantial portion of a breast, and having an outlet through which the nipple passes. In one embodiment of the invention, the funnel-shaped hood has a flexible, i.e. resilient, thin-walled section formed in the conical portion of the hood near the outlet. At the outlet of the hood there is also advantageously provided an elongated flexible sleeve which receives the nipple therein. The sleeve, having both a diameter and a longitudinal axis, communicates at its upstream end with the hood outlet and at its downstream end with a milk flow path to a milk receptacle.

In another embodiment, a rigid frame extends along the exterior of the flexible sleeve and also supports the conical portion of the hood, as well as the flexible sleeve ends. The sleeve is free to contract radially, but not longitudinally.

When an intermittent vacuum (negative pressure) is applied within the hood from a suction device, which may be manually operated or motor driven, the breast is drawn further into and compressed within the hood and sleeve. In a preferred embodiment of the invention, the flexible thin-walled section of the conical portion of the hood is supported by a complimentary shaped ring-like hood mount which is part of the hood frame, so as to allow the thin-walled section to contract along its diameter to compress and gently massage the areola, enhancing milk expression. That is, the thin-walled section of the conical portion of the hood is drawn toward the areola by the suction generated within the hood. The hood mount substantially prevents contraction of the remainder of the flexible hood along its longitudinal axis. The thin-walled section thus intermittently touches or strokes the areola under the influence of the suction pump in an action considered very reminiscent of the suckling of an infant.

In another embodiment, the flexible sleeve extending from the outlet is adapted to contract along its diameter to compress and gently massage the nipple and the adjacent breast, likewise enhancing milk expression. The exterior frame along the sleeve substantially prevents contraction of the sleeve along its longitudinal axis. The sleeve thus intermittently squeezes the nipple and adjacent breast under action of the suction pump, again in an action which is considered to be reminiscent of the suckling of an infant.

The invention further provides an area of stimulation that accommodates nipples and breasts of various sizes and shapes. The areola and nipple massage features can be used separately, or incorporated in a single breast pump.

The present invention, together with its attendant objectives and advantages, will be further understood with reference to the detailed description below read in conjunction with the accompanying drawings, in which:

## BRIEF DESCRIPTION OF THE DRAWINGS

Fig. 1 is a vertical sectional view of part of a first breast pump made in accordance with the present invention;
Fig. 2 shows an exploded elevational view of the hood, including flexible sleeve, and the frame of Fig. 1; and
Fig. 4 is a vertical sectional view of part of a second breast pump made in accordance with the present invention;
Fig. 5 shows an exploded elevational view of the hood and frame of Fig. 4; and

Fig. 6 is a cross-sectional view along the line 6-6 of Fig. 4.

## DETAILED DESCRIPTION OF TWO EMBODIMENTS OF THE INVENTION

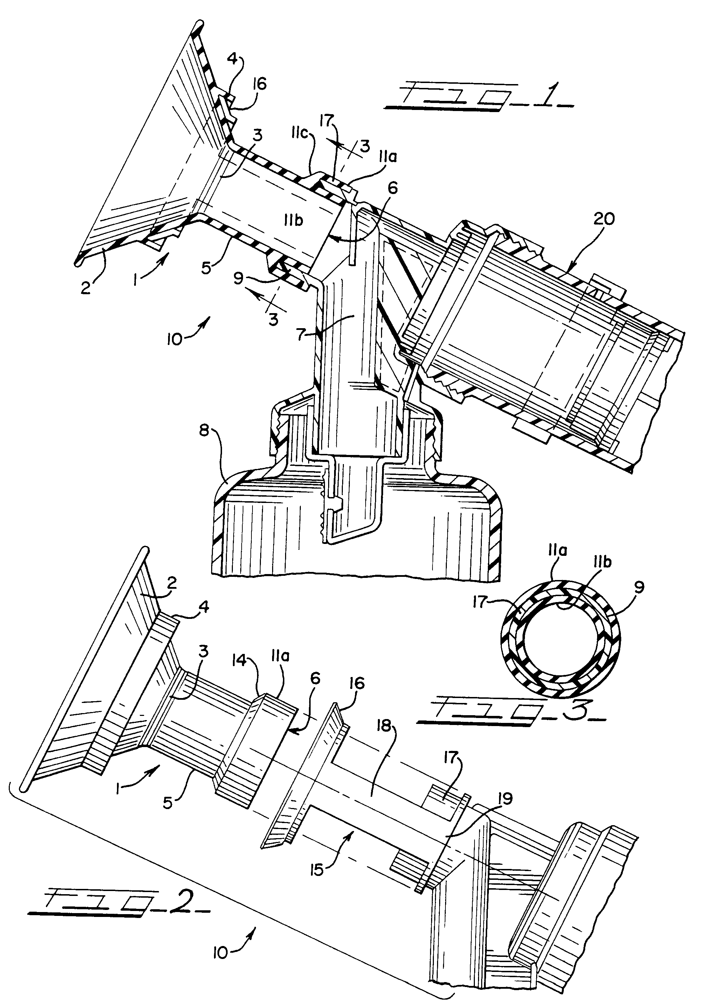

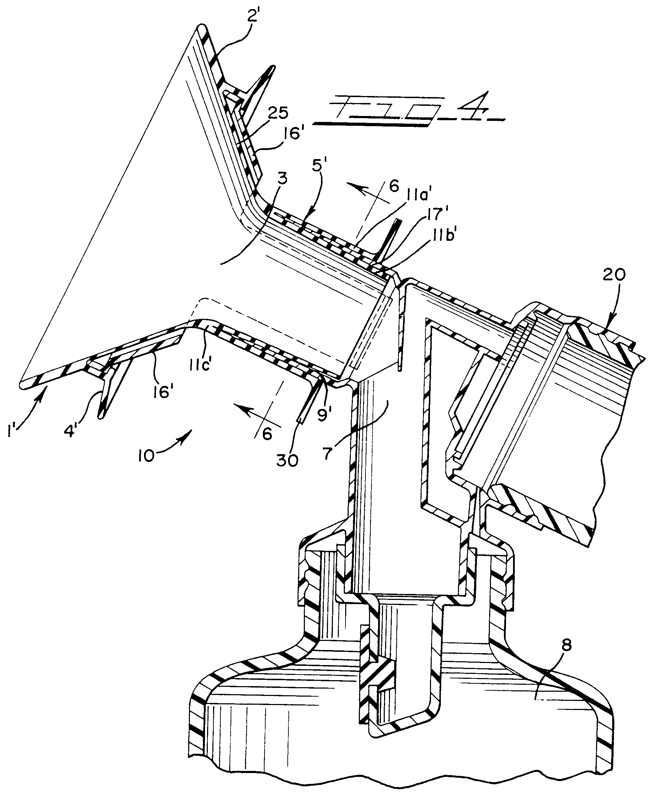

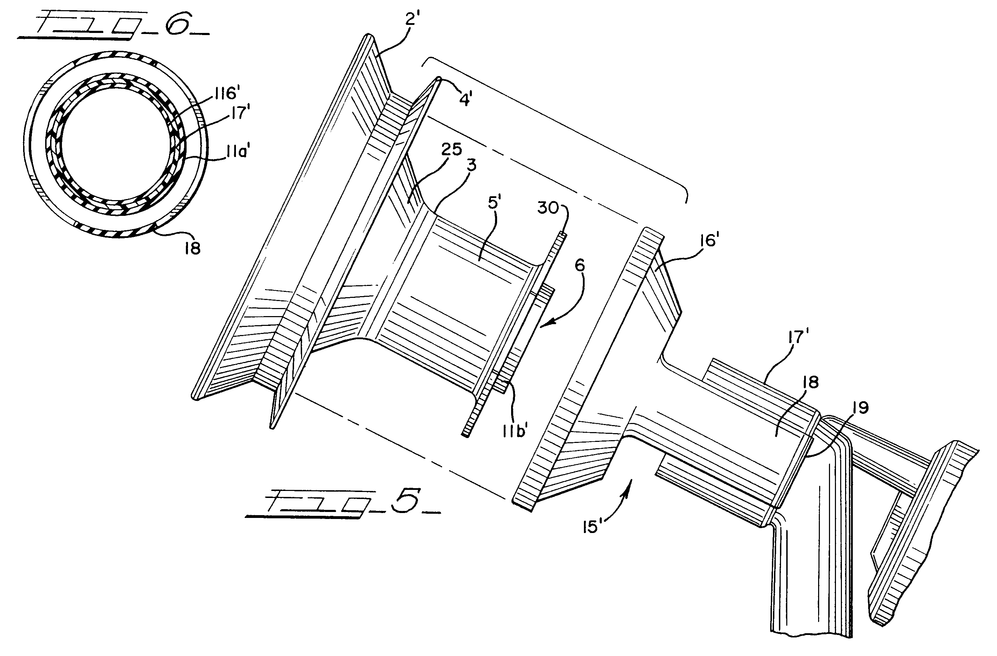

A first embodiment of the invention is illustrated in Figs. 1-3. A breast pump made in accordance with the teachings of the present invention is generally designated at 10. The breast pump 10 has a funnel shaped hood 1. Hood 1 has a forward portion 2 with a conical dish-like shape, which is designed to encompass a substantial portion of the breast centered on the nipple. Downstream end 3 of the hood constitutes an outlet to the foregoing "dish." Along the exterior of conical portion 2 is a mounting lip 4. Use of the mounting lip 4 will be described in detail hereafter.

The outlet 3 opens into an elongated flexible sleeve 5 which extends axially from the outlet 3. The sleeve 5 is tubular with a generally circular radial cross-section along its length. A downstream sleeve end 6 communicates with a milk flow path 7 to a milk receptacle 8, i.e., a milk bottle. Downstream sleeve end 6 also communicates with a means for applying an intermittent vacuum to the flexible sleeve 5 and conical portion 2, such as the manual suction pump 20. Further details of the breast pump can be gleaned from applicant's U.S. Patent No. 4,857,051, and U.S. Patent Application Serial No. 07/278,047, filed November 30, 1988, both of which are incorporated herein by reference.

An annular channel 9 is formed at downstream sleeve end 6 by walls 11a, 11b, which are spaced from, but concentric with, each other. The annular channel 9 is open at its downstream end, and closed at its upstream end by a shoulder 11c which extends between the walls 11a, 11b. The use of this annular channel 9 to mount the sleeve end 6 is described below.

A rigid frame 15 supports the hood member 1 and the flexible sleeve 5. The frame 15, which here is a permanently fixed part of the upper portion of the breast pump 10, has a ring-shaped hood mount 16 at an upstream end upon which the hood portion is received, and a ring-shaped mount or collar 17 at a downstream end upon which downstream sleeve end 6 is received. The hood mount 16 is angled to match the exterior shape of the conical portion 2. Extending between hood mount 16 and collar 17 are frame members, or struts, 18. Two struts 18 are provided in this embodiment on opposite sides of the frame 15. Radially extending ears 19 space the struts 18 from the collar 17. When the conical portion 2 and flexible sleeve 5 are mounted within the frame 15, the struts 18 extend along the opposite sides of the flexible sleeve 5, fixing the hood mount 16 in spaced relation to the collar 17.

Mount 16 is made to engage the mounting lip 4 on hood 1 in a snap-like fit. Collar 17 and the annular channel 9 are likewise complimentarily sized and shaped so that the collar 17 fits within the annular channel 9 in a substantially air-tight engagement. The hood 1 may thus be readily and removable mounted to the frame 15. Sleeve 5 is thereby fixed at both its upstream end (i.e., at the outlet 3), and its downstream end 6 by the hood mount 16 and collar 17, respectively.

In use, the user places conical portion 2 over the breast with the outlet generally centered on the breast nipple, such that the nipple passes through the hood outlet. Intermittent suction from pump 20 causes the breast and nipple to be cyclically drawn further into and compressed within conical portion 2 and sleeve 5. Sleeve 5 intermittently contracts along its diameter to compress and gently massage the nipple and adjacent breast. This gentle squeezing is considered very reminiscent of suckling, and enhances the expression of milk. Frame 15 substantially prevents contraction of sleeve 5 along its longitudinal axis, which could otherwise cause discomfort or inhibit milk flow by pinching the nipple. A fairly long flexible area is also provided by the sleeve 5, which allows the sleeve 5 to accommodate a variety of nipple sizes and shapes.

In this embodiment, hood 1 is molded in an integral piece from a flexible plastic, such as silicone. The overall length of the hood 1 measures about 2.25 inches from the forward rim of the hood portion 2 to the downstream sleeve end 6 (measured along the hood axis). Sleeve 5 is about 1.25 inches in length, with an interior diameter of about 29/32 inch (or 23 millimeters) at its upstream end tapering to about 26/32 inch (or 21 millimeters) at its downstream end 6. The wall thickness of sleeve 5 is a substantially uniform 0.4 millimeters. Hood portion 2 has a fairly uniform thickness of about 1.5 millimeters, from the thickened bead around the forward rim to about the mounting lip 4 (which lip has a thickness of about 0.8 millimeter). The hood portion thickness between lip 4 and sleeve 5 is about 1 millimeter. The inner diameter of annular channel wall 11a is about 33/32 inch (or 26 millimeters).

It should be noted that a variety of other similar means may be provided for mounting the conical portion 2 and sleeve 5 in the intended manner instead of the frame 15 described herein. Conical portion 2 and sleeve 5 also need not be made integral, although a one-piece flexible unit is considered most desirable.

A second embodiment of the invention is illustrated in Figs. 4-6. This embodiment is particularly adapted for massage of the areola, rather than the nipple. Like numbers to those used in describing the first embodiment indicate the same or substantially the same parts in the second embodiment.

As in the first embodiment, a downstream sleeve end 6 communicates with a milk flow path 7 to a milk receptacle 8. Downstream sleeve end 6 also communicates with a means for applying an intermittent vacuum to the hood, such as the manual suction pump 20.

An extended annular channel 9' is formed at downstream sleeve end 6 by walls 11a', 11b', which are concentric and spaced from each other. In this second embodiment, hood 1' is formed of the same flexible plastic as hood 1.

Extending from at least the mounting lip 4' to the outlet 3 is a flexible thin-walled portion 25. A sleeve flange 30 is formed on the downstream end of the sleeve to afford the user a convenient means for attaching the sleeve as well as for removing the sleeve from the frame for cleaning. The annular channel 9' is open at its downstream end, and closed at its upstream end by a shoulder 11c' which extends between the walls 11a' and 11b' in the area at the outlet 3. The use of this annular channel 9' to mount the sleeve 5' is described below.

A rigid frame 15' supports the hood member 1' and the sleeve 5'. The frame 15' has a ring-shaped hood mount 16' at an upstream end upon which the hood conical portion 2' is received, and a longitudinally extended, ring-shaped collar 17' at a downstream end over which sleeve 5' is mounted. The hood mount 16' is angled to match the exterior shape of the conical portion 2'. Extending between hood mount 16' and collar 17' are frame members, or struts, 18. Again, two struts 18 are provided on opposite sides of the frame 15'. Radially extending ears 19 space the struts 18 from the base of collar 17'.

Mount 16' is made to engage the mounting lip 4' on hood 1' in a snap-like fit. As can be seen in Fig. 4, however, the thin-walled section 25 is spaced slightly inwardly from the mount 16'. Collar 17' and the annular channel 9' are likewise complimentary sized and shaped so that the collar 17' fits within the annular channel 9' in a substantially air-tight engagement. In this embodiment, collar 17' tapers to a fairly sharp edge in the vicinity of the outlet 3. The sleeve 5' is thereby supported along substantially its entire length.

With the hood portion 2' and sleeve 5' mounted within the frame 15', frame members 18 extend along opposite sides of the flexible sleeve 5', fixing the hood mount 16' in spaced relation to the collar 17'. The hood 1' is readily and removably mounted to the frame 15'. The radially extended mounting lip 4' provides a good gripping surface, and further facilitates mounting and removal of the hood 1', as does the sleeve flange 30.

The user places hood portion 2' over the breast with the outlet 3 generally centered on the breast nipple, such that the nipple passes through the hood outlet. Intermittent suction from pump 20 causes the nipple and areola to be cyclically drawn further into and compressed within conical portion 2' and sleeve 5'. Thin-walled portion 25 intermittently contracts along portions of its diameter to compress and gently stroke the areola. This gentle stroking is considered very reminiscent of suckling, and enhances the expression of milk. Collar 17' combined with frame 15' substantially prevents contraction of sleeve 5' along its longitudinal axis, which could cause discomfort or inhibit milk flow by pinching the nipple.

In this second embodiment, hood 1' is also molded in an integral piece from a flexible plastic, such as silicone. The overall length of hood 1' measures about 2.25 inches from the forward rim of the hood portion 2' to the downstream sleeve end 6 (measured along the hood axis). Since sleeve 5' is laterally supported along its entire length, and therefore does not radially contract in any significant way, its dimensions are not deemed critical, but are about the same as in the previously described embodiment. Flexible thin-walled portion 25 has a thickness of about .4 millimeter.

Thus, while the invention has been described with reference to certain embodiments, those skilled in the art will recognize modifications of structure, arrangement, composition and the like that can be made to the present invention, yet still fall within the scope of the invention as hereafter claimed.

**Claims**

1. A breast pump for mother's milk comprising:

   a hood having a generally funnel-shape with a conical portion adapted to be applied to and surround a substantial portion of a breast with said hood generally centered on the breast nipple, said hood having a central longitudinal axis, an outlet through which the nipple passes, and a flexible portion overlying the breast tip constituting the nipple and areola area when in use,

   means for receiving and conveying milk expressed from the breast to a milk receptacle,

   means for applying an intermittent vacuum to said hood to cause the breast and nipple to be drawn further within and compressed within said hood for the expression of milk, said flexible portion being drawn toward said central longitudinal axis to compress, massage and stimulate the breast tip area for enhanced milk expression, and

   rigid frame supporting said hood and substantially preventing longitudinal contraction of said hood along said central longitudinal axis when said hood is under vacuum.

2. A breast pump for mother's milk comprising:

   a hood having a generally funnel-shape with a conical portion adapted to be applied to and surround a substantial portion of a breast generally centered on the breast nipple, said conical portion having an outlet through which the nipple passes, and a sleeve which is elongated and flexible, said sleeve having a longitudinal axis, a diameter across said axis, an upstream sleeve and extending from said outlet and a downstream sleeve end communication with a milk flow path to a milk receptacle, said sleeve receiving the nipple therein,

   a rigid frame supporting said sleeve having, a ring-shaped collar at a downstream end upon which said downstream sleeve end is received in substantially air-tight engagement therewith, and a rigid frame member extending between and spacing apart said conical portion and said ring-shaped collar, said sleeve being received within said rigid frame, and

   means for applying an intermittent vacuum to said hood to cause the breast and nipple to be drawn further within and compressed within said hood including said sleeve for the expression of milk, said sleeve contracting along its diameter to compress, massage and stimulate the nipple and adjacent breast for enhanced milk expression without contraction of said sleeve longitudinally along said longitudinal axis.

3. A breast pump for mother's milk comprising:

   a flexible hood having a generally funnel-shape, including a conical portion adapted to be applied to and surround a substantial portion of a breast generally centered on the breast nipple, said hood having a hood outlet through which the nipple passes, and an elongated flexible sleeve extending from said hood outlet, said elongated flexible sleeve having a generally circular axial cross-section, a downstream sleeve end communicating with a milk flow path to a milk receptacle, a flexible wall formed concentric with said sleeve at said downstream sleeve end, said wall being spaced from and surrounding said downstream sleeve end to thereby define an annular channel with said elongated flexible sleeve which channel is open at a downstream channel end and closed at an upstream channel end by a shoulder extending between said flexible wall and said elongated flexible sleeve wall,

   a rigid frame supporting said conical portion and elongated flexible sleeve, said rigid frame having a ring-shaped hood mount at an upstream frame end upon which ring-shaped hood mount said conical portion is received, a ring-shaped collar at a downstream frame end upon which said downstream sleeve end is received, said ring-shaped collar and said annular channel being complimentarily sized so that said ring-shaped collar fits within said annular channel in a substantially air-tight engagement to thereby removably mount said elongated flexible sleeve on said rigid frame, and a rigid frame member exterior to and extending along said elongated flexible sleeve and fixing said ring-shaped hood mount and ring-shaped collar in spaced relationship, and

means for applying an intermittent vacuum to said elongated flexible sleeve and funnel-shaped hood to cause the breast and nipple to be drawn further within and compressed within said hood including said elongated flexible sleeve for the expression of milk, said elongated flexible sleeve contracting along its diameter to compress and gently massage the nipple and adjacent breast for enhanced milk expression, said rigid frame substantially preventing contraction of said elongated flexible sleeve along a longitudinal sleeve axis.

4. The breast pump of claim 3 wherein said elongated flexible sleeve defines an area of stimulation which has an axial length of at least 1.25 inches extending from said hood outlet, and an interior diameter of about 26/32 inch.

5. The breast pump of claim 4 wherein said hood, including elongated flexible sleeve, is molded in an integral piece.

6. A breast pump for mother's milk comprising:
a hood having a generally funnel-shape with a conical portion adapted to be applied to and surround a substantial portion of a breast generally centered on the breast nipple, said hood having a central axis, and an outlet through which the nipple passes, said outlet being in fluid communication with a flow path to a milk receptacle, said conical portion having a flexible thin-walled section overlying the area of the areola when the breast pump is in use,

a rigid frame supporting said hood, said rigid frame having a ring-shaped hood mount at an upstream end and upon which ring-shaped hood mount said conical portion is received, a second mount at a downstream end which fixes said hood in place in the area of said outlet, and a rigid interconnecting frame member extending between and spacing apart said ring-shaped hood mount and second mount, with said flexible thin-walled section located between said ring-shaped hood mount and said second mount,

means for applying an intermittent vacuum to said hood to cause the breast and nipple to be drawn further within and compressed within said hood for expression of milk, said flexible thin-walled section being drawn toward said central axis to compress, massage and stimulate the nipple and adjacent breast for enhanced milk expression without contraction of said conical portion longitudinally along said central axis.

7. A breast pump for mother's milk comprising:
a hood which is flexible and generally funnel-shaped with a conical portion adapted to be applied to and surround a substantial portion of a breast, with said hood generally centered on the breast nipple, said hood having a central axis, a resilient thin-walled section overlying the area of the areola when in use and sized to encompass substantially all of the areola, and an outlet through which the nipple passes,

a flexible sleeve extending from said hood outlet with a downstream sleeve and communicating with a milk flow path to a milk receptacle, said flexible sleeve receiving the nipple therein,

a rigid frame supporting said hood, said rigid frame having a ring-shaped hood mount at an upstream end upon which said ring-shaped hood mount said conical portion is received, said ring-shaped hood mount fixing said conical portion in place at a point upstream from said resilient thin-walled section, and a second mount located downstream from said hood outlet upon which said downstream sleeve end is received to fix said downstream sleeve end in place, and

means for applying an intermittent vacuum to said hood to cause the breast and nipple to be drawn further within and compressed within said hood for the expression of milk, said resilient thin-walled section contracting toward said central axis to gently massage the areola for enhanced milk expression.

8. A breast pump for mother's milk comprising:
a hood having a generally funnel-shape with a flexible conical portion adapted to be applied to and surround a substantial portion of a breast generally centered on the breast nipple, said funnel-shaped flexible hood having a central axis, an outlet through which the nipple passes, a resilient thin-walled section which is ring-shaped and formed in said conical portion overlying the area of the areola when in use and sized to encompass substantially all of the areola,

an elongated flexible sleeve extending from said outlet, said elongated flexible sleeve having a downstream end communicating with a milk flow path to a milk receptacle, a flexible wall formed concentric with said sleeve, said wall being spaced from and surrounding said downstream sleeve end to thereby define an annular channel with said elongated flexible sleeve which is open at a downstream end and closed at an upstream end between said flexible wall and said elongated flexible sleeve,

a rigid frame supporting said hood, said rigid frame having a ring-shaped hood mount at an upstream end upon which ring-shaped hood mount said conical portion is received, said ring-shaped hood mount fixing said conical portion in place at a point upstream from said resilient thin-walled section, a ring-shaped collar at a downstream end upon which said downstream sleeve end is received, said collar and said annular channel being complimentarily sized so that said ring-shaped collar fits within said annular channel in a substantially air-tight engagement to thereby removably mount said flexible sleeve on said rigid frame, said ring-shaped collar extending to about said outlet, said rigid frame member fixing said ring-shaped hood mount and ring-shaped collar in spaced relationship, and

means for applying an intermittent vacuum to said hood to cause the breast and nipple to be drawn further within and compressed within said conical portion and elongated flexible sleeve for the expression of milk, said resilient thin-walled section contracting along its diameter and toward said central axis to gently massage the areola for enhanced milk expression, said rigid frame substantially preventing longitudinal contraction of said elongated flexible sleeve and said conical portion along said central axis.

9. The breast pump of claim 8 wherein said elongated flexible sleeve has a radially outwardly extending flange formed thereon sized for gripping by a user to facilitate mounting and demounting of said hood.

10. The breast pump of claim 9 wherein said hood, including said conical portion and elongated flexible sleeve, is molded in an integral piece.

11. The breast pump of claim 8 wherein said conical portion has a radially outwardly extending lip formed thereon surrounding said conical portion in the area where said conical portion is fixed in place on said ring-shaped hood mount, said lip being sized for use in hand-mounting and demounting of said conical portion.

12. A breast pump for mother's milk comprising:
a hood having a generally funnel-shaped hood including a conical portion adapted to be applied to and surround a substantial portion of a breast with said hood generally centered on the breast nipple, said hood having a central longitudinal axis, an outlet through which the nipple passes which outlet is in fluid communication with means for receiving and conveying milk from the breast to a milk receptacle, said hood further having a flexible portion overlying the area of the areola when in use,
a rigid frame supporting said hood, said rigid frame including an upper mount upon which said conical portion is received and which serves to fix said conical portion in place on said rigid frame, said rigid frame further including a lower mount for fixing said conical portion in place located adjacent said outlet, said flexible portion being positioned between said upper and lower mounts, and

means for applying an intermittent vacuum within said hood to cause the breast and nipple to be drawn further within and compressed within said hood for the expression of milk, said flexible portion being drawn toward said central axis to compress and massage the areola and adjacent breast for enhanced milk expression, said upper and lower mounts substantially preventing longitudinal contraction of any part of said conical portion along said axis and toward said outlet when said hood is under vacuum.

13. A breast pump for mother's milk comprising:
a hood having a generally funnel-shape with a conical portion adapted to be applied to and surround a substantial portion of a breast with said hood generally centered on the breast nipple, said hood having a central longitudinal axis, an outlet through which the nipple passes, and a thin-walled flexible portion overlying substantially only the area of the areola when in use,
means for restraining substantially any movement of said conical portion in a direction parallel to said central longitudinal axis while allowing contraction of said thin-walled flexible portion radially toward said central longitudinal axis, and
means for applying an intermittent vacuum to said hood to cause the breast and nipple to be drawn further within and compressed within said hood for the expression of milk, said thin-walled flexible portion being drawn toward said central longitudinal axis under influence of said vacuum to compress and massage the areola for enhanced milk expression.

14. A breast pump for mother's milk comprising:
a hood having a flexible generally funnel-shape and including a conical portion adapted to be applied to and surround a substantial portion of a breast with said hood generally centered on the breast nipple, said hood having a longitudinal axis, an outlet through which the nipple passes, and a resilient portion overlying the tip of the breast constituting the nipple and areola when in use,
means for receiving and conveying milk expressed from the breast to a milk receptacle,
means for applying an intermittent vacuum to said hood to cause the breast and nipple to be drawn further within and compressed within said hood for the expression of milk, said resilient portion being drawn toward said longitudinal axis to compress and massage the tip of the breast for enhanced milk expression under influence of vacuum, and

a frame which is rigid supporting said hood and substantially preventing longitudinal contraction of any portion of said hood along said axis when said hood is under vacuum, said frame having a hood mount located upstream from said resilient portion and a hood mount located downstream from said resilient portion, with frame member means for interconnecting said hood mounts in fixed spaced relationship within said resilient portion located therebetween.

15. The breast pump of claim 14 wherein said resilient portion is located only in a ring-shaped region of said conical portion which overlies the areola in use.

16. The breast pump of claim 14 wherein said resilient portion is located only in an elongated sleeve extending from said outlet within which sleeve the nipple is received.

17. A breast pump for mother's milk comprising:

a hood having a generally funnel-shape with a conical portion adapted to be applied to and surround a substantial portion of a breast with said hood generally centered on the breast nipple, said hood having a longitudinal axis which is generally central to an outlet through which the nipple passes,

said hood further having a resilient portion overlying substantially only a desired area of the breast tip constituting the nipple and areola when in use,

means for applying an intermittent vacuum to said hood to cause the breast and nipple to be drawn further within and compressed within said hood for the expression of milk, said resilient portion being drawn toward said longitudinal axis under the influence of vacuum to gently massage the desired area of the breast tip for enhanced milk expression, and

means for mounting said resilient portion to restrain said resilient portion against any substantial movement in a direction parallel to said longitudinal axis, said resilient portion being the only part of said hood that is movable toward said longitudinal axis.

FIG_1

FIG_2

FIG_3

_FIG_4_

_FIG_6_

_FIG_5_

EP 0 466 462 A1

**European Patent Office**

**EUROPEAN SEARCH REPORT**

Application Number

EP 91 30 6228

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | DE-U-8 812 030 (KA WE CO GMBH)<br>* page 4, line 1 - page 5, line 6 *<br>* page 8, line 1 - page 10, line 9 *<br>* figures 1,2 * | | A61M1/06 |
| X | | 1-3,<br>6-10,<br>12-17 | |
| A | | 5 | |
| | --- | | |
| A | FR-A-1 067 421 (A.DURAND ET AL)<br>* the whole document * | ALL | |
| | --- | | |
| X | US-A-4 607 596 (W.WHITTLESTONE ET AL.)<br>* column 3, line 9 - line 48 *<br>* figures 1,2 * | 1 | |
| | --- | | |
| A | US-A-2 542 505 (G.GASCOGNE)<br>* the whole document * | 1 | |
| | ----- | | |
| | | | TECHNICAL FIELDS SEARCHED (Int. Cl.5) |
| | | | A61M |

The present search report has been drawn up for all claims

| Place of search | Date of completion of the search | Examiner |
|---|---|---|
| THE HAGUE | 04 OCTOBER 1991 | VEREECKE A. |

EPO FORM 1503 03.82 (P0401)